## Europäisches Patentamt

## European Patent Office

## Office européen des brevets

(11) Numéro de publication: **0 086 147**

**B1**

(12) **FASCICULE DE BREVET EUROPÉEN**

(45) Date de publication du fascicule du brevet:
**29.04.87**

(21) Numéro de dépôt: **83400218.0**

(22) Date de dépôt: **02.02.83**

(51) Int. Cl.⁴: **A 61 F 2/78**

(54) **Dispositif fixe-prothèse.**

(30) Priorité: **05.02.82 FR 8201855**

(43) Date de publication de la demande:
**17.08.83 Bulletin 83/33**

(45) Mention de la délivrance du brevet:
**29.04.87 Bulletin 87/18**

(84) Etats contractants désignés:
**AT BE DE GB IT SE**

(56) Documents cité:
**BE-A-675 386**
**FR-A-1 135 516**
**FR-A-1 532 625**
**FR-E-71 219**
**FR-E-73 157**
**US-A-3 600 717**

(73) Titulaire: **Belzidsky, David, 191 rue Saint Charles,
F-75015 Paris (FR)**

(72) Inventeur: **Belzidsky, David, 191 rue Saint Charles,
F-75015 Paris (FR)**

(74) Mandataire: **Viard, Jean, Cabinet VIARD 28 bis,
avenue Mozart, F-75016 Paris (FR)**

LIBER, STOCKHOLM 1987

## Description

L'invention concerne un dispositif spécialement adapté à la protection de membres amputés et au port de prothèse.

On connait déjà par FR-A-1 135 516 un ensemble de protection à l'usage des amputés utilisant des appareils prothétiques. Cet ensemble comprend une gaine fine, à faible coefficient de frottement, cette gaine de contact se plaçant sur le membre amputé ce grâce à quoi on réduit les inflammations, écorchures et toutes les causes résultant du frottement occasionné par le port d'un appareil de prothèse, en particulier dans le cas d'amputation d'un membre inférieur.

On connait également par FR-E-71 219, l'usage d'un combiné formé d'une gaine de contact en nylon et d'un bonnet couvre-moignon, par exemple en laine, chaussé par dessus la gaine de sorte que le membre amputé est toujours protégé par la gaine fine qui peut glisser dans le bonnet de laine mais ce dernier présente, extérieurement, un coefficient de frottement supérieur à la gaine ce qui permet de le stabiliser par rapport aux parois d'une prothèse. Ainsi la protection se trouve accrue, le confort amélioré. Pour éviter qu'à l'usage la gaine de contact forme des plis blessants, on assure la fixation de la gaine de contact et du bonnet couvre moignon en rabattant gaine et bonnet sur le bord supérieur de la prothèse. Ce mode de fixation empêche le glissement de la gaine de contact sur le membre amputé et la formation de plis blessants.

Bien que cet ensemble constitue un progrès notable dans le port des prothèses, notamment les prothèses pour amputation de jambe sans cuissard, l'usage montre toutefois, avec ce mode de fixation connu qui vient d'être décrit, un phénomène de léger pompage de la gaine de contact et du bonnet dans le manchon de la prothèse dû au fait que le membre est plus ou moins bien assujetti à la prothèse malgré les rabats de fixation sur la prothèse. C'est ainsi que dans la marche, la montée d'escaliers, la montée en voiture, il se produit à chaque mouvement d'élévation du membre inférieur amputé un mouvement relatif de désemboîtement qui, à la longue, cause un inconfort, fatigue le membre amputé toujours sensible aux mouvements alternatifs de pompage par rapport à la prothèse et peut causer des irritations et écorchures.

La présente invention a en conséquence pour but principal un dispositif assurant à la fois la protection épidermique d'un membre amputé, notamment un membre inférieur, et la fixation correcte du membre protégé par rapport à une prothèse.

Un autre but de l'invention est de fournir un dispositif de protection et de fixation qui supprime pratiquement dans le port de prothèse l'effet de pompage difficile à supporter.

Un autre but encore est de fournir un dispositif qui améliore très sensiblement la tolérance du port des prothèses.

Ces différents buts sont atteints par le dispositif selon l'invention, c'est-à-dire un dispositif fixe-prothèse pour la protection et la fixation d'un membre amputé dans une prothèse à manchon amovible, l'extrémité supérieure du manchon, qui est introduit dans la prothèse et qui fait saillie au dessus de la prothèse, constituant le bord de l'ouverture supérieure de l'emboîture de la prothèse, ce dispositif comprenant une première gaine fine de contact avec le membre amputé recouverte par un bonnet couvre-moignon, et étant caractérisé en ce qu'il comprend en outre une seconde gaine fine couvre-bonnet à faible coefficient de frottement venant au contact interne du manchon et une troisième gaine fine couvre-manchon à faible coefficient de frottement venant à l'intérieur de la cavité d'emboîtement du membre amputé dans la prothèse, et une gaine fixe-prothèse en tissu élastique, ouverte à ses deux extrémités, chaussant la prothèse et le membre protégé, et présentant au voisinage de ses deux extrémités ouvertes respectivement une zone de fixation élastique supérieure et inférieure et une zone intermédiaire à propriété élastique différente, ladite gaine fixe-prothèse venant à recouvrement partiel sur la partie supérieure de la première gaine fine de contact, du bonnet, de la seconde gaine fine couvre-bonnet et de la troisième gaine fine couvre-manchon, cesdites parties supérieures dépassant la zone supérieure de la gaine fixe-prothèse d'une longueur telle qu'elles peuvent être repliées une première fois par rabat simple sur l'extrémité de la zone supérieure de la gaine fixe-prothèse tout en laissant la possibilité d'effectuer un second pli par rabat en commun avec ladite extrémité de la zone supérieure pour former un bourrelet de fixation élastique plat dont le bord inférieur vient en butée contre le bord de l'ouverture supérieure de l'emboîture de la prothèse.

Le bord de la gaine fixe-prothèse emprisonné dans le double rabat du bourrelet de fixation garantit la tension permanente des gaines et bonnet.

La seconde gaine couvre-bonnet assure au niveau de l'articulation un déplacement angulaire relatif entre la première gaine fine et son bonnet par rapport au manchon d'emboîtement.

La troisième gaine couvre-manchon assure le glissement d'emboîtement du manchon dans l'emboîture de la prothèse.

Le dispositif d'ensemble immobilise axialement le membre amputé par rapport à la prothèse mais autorise et favorise les mouvements angulaires au niveau de l'articulation, les deuxième et troisième gaines faisant fonction de palier glissant.

Suivant une disposition, la gaine élastique fixe-prothèse a ses deux zones d'extrémité inextensibles axialement mais élastiquement extensibles radialement.

Suivant une autre disposition, la gaine élastique fixe-prothèse a sa zone intermédiaire qui présente une double extensibilité, l'une

relativement faible dans le sens axial, l'autre plus extensible dans le sens radial, au niveau de l'articulation du membre protégé.

D'autres dispositions apparaitront encore dans la description détaillée qui suit d'un mode de réalisation donné ici à titre d'exemple dans les dessins annexés dans lesquels:

la Figure 1 est une vue en coupe montrant les différents composants du dispositif à l'état chaussé, en relation d'assemblage avec une prothèse de jambe du genre à manchon d'emboîtement,

la Figure 2 est une vue en coupe partielle, semblable à la figure 1 montrant la première phase d'assemblage des composants du dispositif,

la Figure 3 est une vue en coupe partielle semblable à la figure 1 montrant la seconde phase de fixation des composants par rapport à la prothèse,

la Figure 4 est une vue du fixe-prothèse du dispositif à l'état monté sur la prothèse, avant fixation.

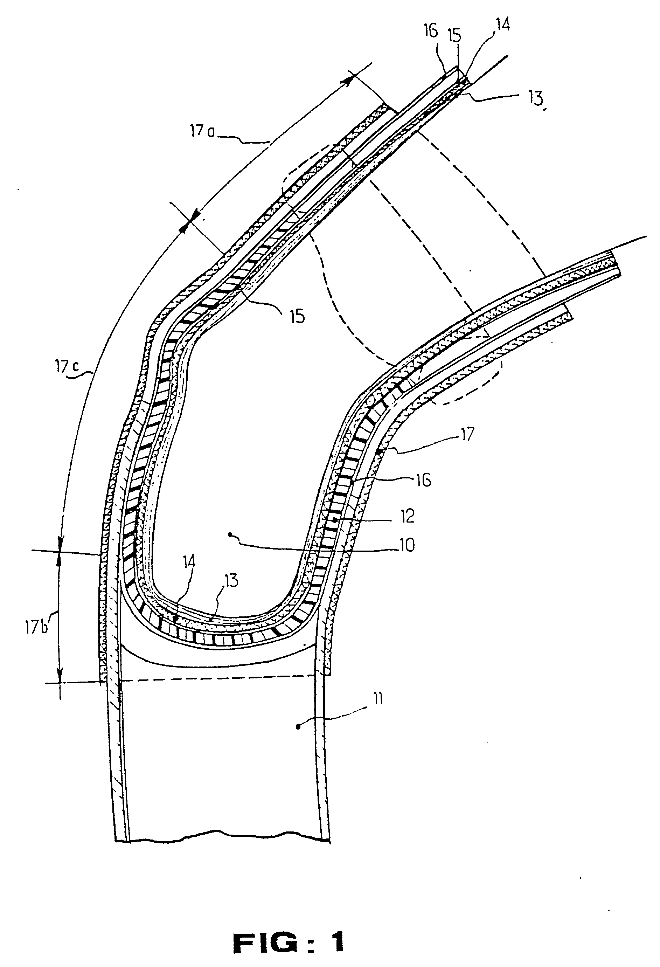

Dans l'exemple d'exécution de la figure 1 on a montré l'application du dispositif au port d'une prothèse à un membre inférieur amputé désigné par la référence 10. Dans ce cas, l'amputation est située sensiblement au dessous du genou.

La prothèse 11 est du type à manchon emboîtable 12 en soi connu par exemple en élastomère moulé.

Le dispositif pour la protection épidermique du membre amputé comprend tout d'abord une première gaine de contact fine 13 tricotée à la manière d'un bas, dans un fil synthétique fin traité spécialement pour garantir une absence totale de réaction au contact de l'épiderme. La première gaine est recouverte d'un bonnet couvre-moignon 14 par exemple un bonnet de laine constituant un matelas amortisseur de chocs mais aussi matelas absorbant pour la transpiration, ce grâce à quoi on élimine une des causes d'irritation du membre amputé protégé. Dans le cas d'une prothèse de jambe à emboîture comme il est montré figure 1, on a trouvé avantageux d'avoir recours à une seconde gaine fine 15 ou gaine couvre-bonnet, à faible coefficient de frottement, recouvrant le bonnet 14 et qui présente une double fonction ; la gaine 15 permet l'emboîtement aisé du manchon de maintien en élastomère 12 par dessus la gaine de contact 13 et le bonnet couvre-moignon 14. On comprend que par cette disposition, la gaine 15 se comporte à la manière d'un palier entre le bonnet couvre-moignon et la surface interne du manchon en élastomère 12 ce qui rend beaucoup plus aisé les mouvements angulaires de pliage lors du jeu de l'articulation au niveau du genou.

Le manchon 12 est chaussé d'une troisième gaine fine 16 à faible coefficient de frottement, semblable aux deux premières et ayant pour fonction de faciliter l'introduction du manchon 12 dans l'emboîture de la prothèse 11 et également de favoriser les mouvements angulaires d'articulation entre le manchon et la prothèse proprement dite.

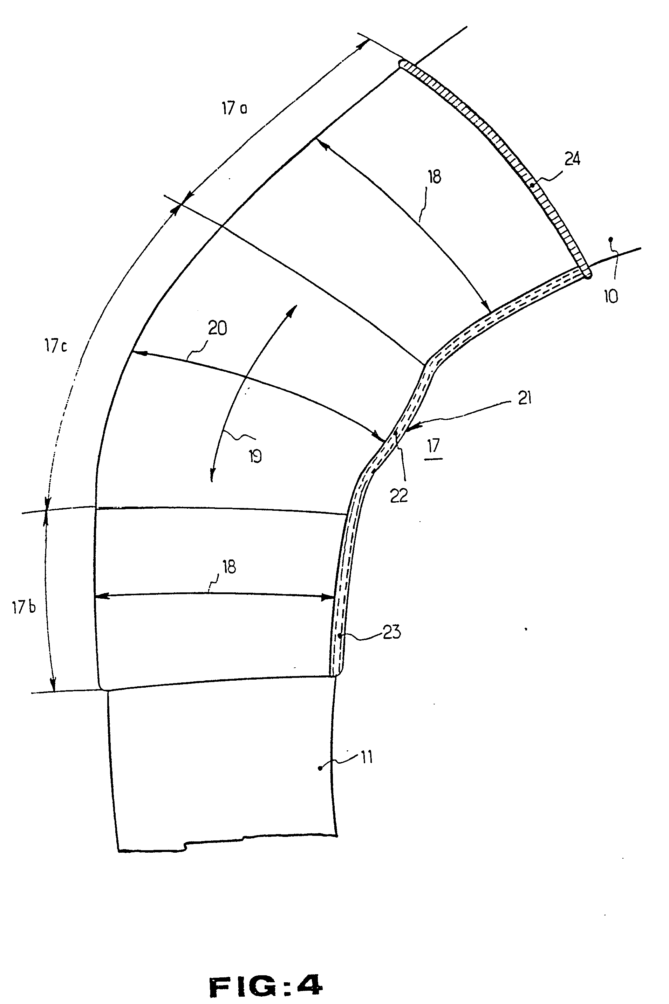

Le dispositif comprend encore une gaine fixe-prothèse 17 en tissu essentiellement élastique se fixant par serrage élastique sur le corps de la prothèse 11. La gaine fixe-prothèse est ouverte à ses deux extrémités et elle présente une zone de fixation élastique supérieure 17a, une zone de fixation élastique inférieure 17b et une zone intermédiaire d'articulation 17c. Le mode d'exécution de la gaine fixe-prothèse sera décrit plus spécialement en relation avec l'exemple de la figure 4.

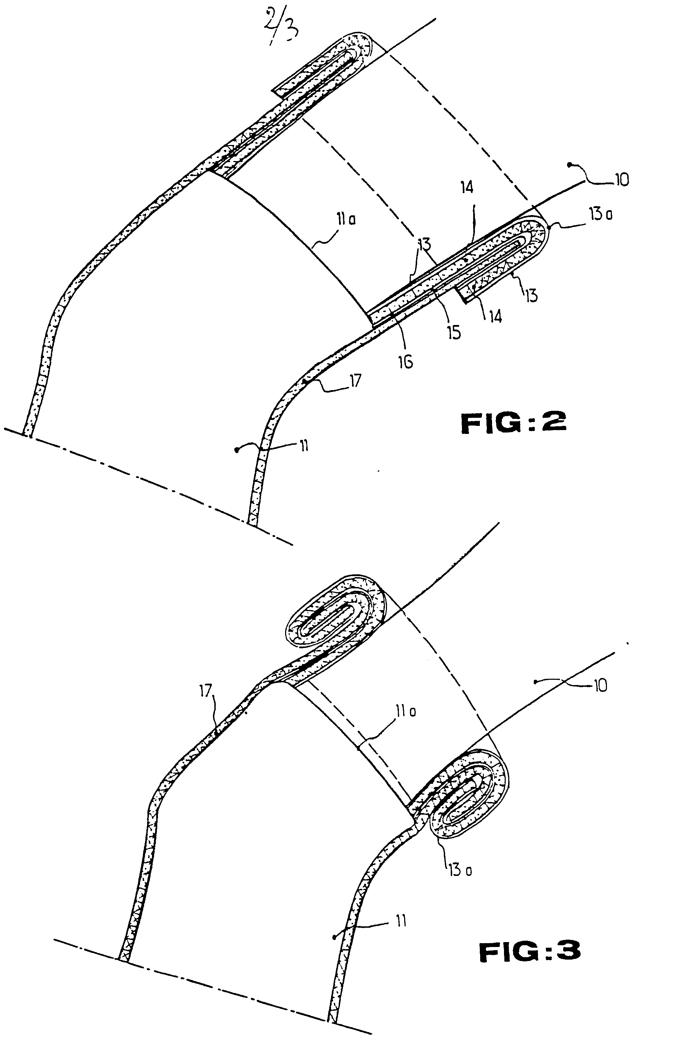

On remarque que la gaine de contact 13, le bonnet 14 et les deux gaines 15 et 16 sont sensiblement plus longues que la gaine élastique fixe-prothèse 17 ce qui permet, comme il est montré aux figures 2 et 3, l'assemblage des composants du dispositif et leur fixation par rapport au bord 11a de l'ouverture supérieure de l'emboîture de la prothèse.

Dans l'exemple de la Figure 2, on replie une première fois la première gaine 13, le bonnet couvre-moignon 14, les gaines 15 et 16 sur l'extrémité de la zone de fixation élastique supérieure 17a de la gaine fixe-prothèse 17. On forme ensuite un second rabat de fixation comme il est montré Figure 3, lequel forme avec le premier rabat un bourrelet de serrage dont l'un des bords 13a vient au contact du bord 11a de l'ouverture de l'emboîture de la prothèse 11. On comprend que par cette disposition, les composants du dispositif sont assemblés entre eux, les gaines et le bonnet couvre-moignon sont tendus, et le bourrelet venant en butée contre le bord de l'ouverture supérieure de l'emboîture de la prothèse, on interdit tout déplacement axial de la prothèse par rapport au membre protégé.

Pour obtenir les fonctions d'assemblage, de fixation et de mouvements relatifs notamment de pliage au niveau de l'articulation, la gaine 17 présente une structure particulière qui est détaillée en figure 4.

La gaine élastique fixe-prothèse 17 a tout d'abord comme particularité le fait que ses deux zones d'extrémité respectivement 17a-17b sont inextensibles axialement mais élastiquement extensibles radialement conformément à la direction des flèches 18. La zone intermédiaire 17c de la gaine fixe-prothèse présente une double extensibilité, l'une relativement faible dans le sens axial et matérialisée par la flèche 19, l'autre plus extensible dans le sens radial et matérialisée par la flèche 20 au niveau de l'articulation du membre protégé. On comprend, que par cette élasticité différenciée dans le sens axial et radial, la partie intermédiaire 17c de la gaine s'adapte aux déformations naturelles d'une articulation notamment lors du pliage. Pour favoriser ce pliage, il est prévu toujours dans la zone intermédiaire 17c sur la génératrice postérieure 21 au niveau de la pliure de l'articulation une légère augmentation de diamètre se traduisant sur le dessin par une portion convexe 22 ce qui favorise lors du pliage de l'articulation la formation d'un pli externe qui

ne gêne en rien la fonction de pliage.

La gaine fixe-prothèse 17 présente tout au long de sa génératrice postérieure 21 une bande pratiquement inextensible 23 formée par exemple d'une bande de tissu inextensible cousue au long de cette génératrice et qui a pour fonction de bloquer les possibilités d'extension de cette gaine lors des mouvements de marche, en particulier dans le sens postéro-antérieur.

On notera encore que la gaine fixe-prothèse 17 présente un bourrelet de repérage 24 au bord de son ouverture supérieure.

L'ensemble de ce dispositif présente un degré de bien-être jamais atteint dans le port des prothèses en particulier celles destinées aux membres inférieurs en raison d'une part du coefficient de frottement extrêmement faible au niveau de l'articulation et d'autre part en raison de l'absence d'effet de pompage.

## Revendications

1. Dispositif fixe-prothèse pour la protection et la fixation d'un membre amputé (10) dans une prothèse (11) à manchon amovible (12), l'extrémité supérieure du manchon (12) qui est introduit dans la prothèse et qui fait saillie au dessus de la prothèse (11) constituant le bord (11a) de l'ouverture supérieure de l'emboîture de la prothèse (11), ce dispositif comprenant une première gaine fine (13) de contact avec le membre amputé (10) recouverte par un bonnet (14) couvre-moignon, caractérisé en ce qu'il comprend en outre une seconde gaine fine (15) couvre-bonnet à faible coefficient de frottement au contact interne du manchon (12) et une troisième gaine fine (16) couvre-manchon à faible coefficient de frottement venant à l'intérieur de la cavité d'emboîtement du membre amputé (10) dans la prothèse (11), et une gaine fixe-prothèse (17) en tissu élastique, ouverte à ses deux extrémités, chaussant la prothèse (11) et le membre protégé (10), et présentant au voisinage de ses deux extrémités ouvertes respectivement une zone de fixation élastique supérieure (17a) et inférieure (17b) et une zone intermédiaire (17c) à propriété élastique différente, ladite gaine fixe-prothèse (17) venant à recouvrement partiel sur la partie supérieure de la première gaine fine de contact (13), du bonnet (14), de la seconde gaine fine couvre-bonnet (15) et de la troisième gaine fine couvre-manchon (16), cesdites parties supérieures dépassant la zone supérieure (17a) de la gaine fixe-prothèse (17) d'une longueur telle qu'elles peuvent être repliées une première fois par rabat simple sur l'extrémité de la zone supérieure (17a) de la gaine fixe-prothèse (17) tout en laissant la possibilité d'effectuer un second pli par rabat en commun avec ladite extrémité de la zone supérieure (17a) pour former un bourrelet de fixation élastique plat dont le bord inférieur (13a) vient en butée contre le bord (11a) de l'ouverture supérieure de l'emboîture de la prothèse (11).

2. Dispositif selon la revendication 1 caractérisé en ce que la gaine élastique (17) fixe-prothèse a ses deux extrémités (17a-17b) inextensibles axialement mais élastiquement extensibles radialement.

3. Dispositif selon les revendications 1 et 2 caractérisé en ce que la gaine élastique (17) fixe-prothèse a sa zone intermédiaire (17c) qui présente une double extensibilité, l'une relativement faible dans le sens axial (19), l'autre plus extensible dans le sens radial (20) au niveau de l'articulation du membre protégé.

4. Dispositif selon l'une quelconque des revendications 1 à 3 caractérisé en ce que la gaine fixe-prothèse (17) présente dans sa zone intermédiaire (17c) sur sa génératrice postérieure (21), au niveau de la pliure de l'articulation une augmentation de diamètre (22) facilitant la fonction de pliage.

5. Dispositif selon l'une quelconque des revendications 1 à 4 caractérisé en ce que la gaine fixe-prothèse (17) présente tout au long de sa génératrice postérieure (21) une bande (23) pratiquement inextensible ayant pour fonction de bloquer l'amplitude de l'extension lors des mouvements de marche dans le sens postéro-antérieur.

6. Dispositif selon l'une quelconque des revendications 1 à 5 caractérisé en ce que la gaine fixe-prothèse (17) présente un bourrelet de repérage (24) au bord de son ouverture supérieure.

## Patentansprüche

1. Vorrichtung zum Befestigen einer Prothese zum Schützen und zum Festlegen eines amputierten Gliedmaßes (10) in einer Prothese (11) mit abnehmbarer Muffe (12), deren oberes Ende, das in die Prothese eingeführt ist und oberhalb der Prothese (11) einen Ansatz bildet, den Rand (11a) der oberen Öffnung des Gelenkes der Prothese (11) bildet, wobei die Vorrichtung zum Kontakt mit dem amputierten Gliedmaß (10) einen ersten dünnen Überzieher (13) enthält, der mit einer Stummelabdeckkappe (14) verdeckt ist, dadurch gekennzeichnet, daß sie außerdem zum Abdecken der Kappe einen zweiten, dünnen, einen geringen Reibungskoeffizienten aufweisenden, mit dem Innern der Muffe (12) in Kontakt kommenden Überzieher (15), zum Abdecken der Muffe einen dritten Überzieher (16) mit geringem Reibungskoeffizienten, der in das Innere des Hohlraums zur Aufnahme des amputierten Gliedmaßes (10) in der Prothese (11) kommt, und einen an beiden Enden offenen Prothesenbefestigungsüberzieher (17) aus elastischem Gewebe enthält, der die Prothese (11) und das gestützte Gliedmaß (10) umhüllt und der im Bereich seiner beiden offenen Enden jeweils eine obere (17a) und eine untere (17b) elastische Befestigungszone und eine Zwischenzone (17c) mit abweichenden elastischen Eigenschaften aufweist, wobei der Prothesenbefestigungsüberzieher (17) zum Teil den oberen Bereich des ersten dünnen Überziehers (13) der Kappe (14) des zweiten die Kappe überdeckenden Überziehers (15) und den dritten die Muffe abdeckenden Überzieher (16) bedeckt, wobei diese oberen Bereiche die obere Zone (17a) des Prothesenbefestigungsüberziehers (17) mit einer derartigen Länge überragen, daß sie ein erstes Mal durch ein einmaliges Umschlagen auf die obere Zone (17a) des Prothesenbefestigungsüberziehers (17) umgeschlagen werden können, wobei die Möglichkeit belassen ist für eine zweite Faltung durch gemeinsames Umschlagen mit besagtem Ende der oberen Zone (17a), um einen flachen, elastischen Haltewulst zu bilden, dessen unterer Rand (13a) an dem Rand (11a) der oberen Öffnung des Gelenkes der Prothese (11) zur Anlage kommt.

2. Vorrichtung nach Anspruch 1, dadurch gekennzeichnet, daß der Prothesenbefestigungsüberzieher (17) an seinen beiden Enden (17a, 17b) axial nicht dehnbar, jedoch radial elastisch dehnbar ist.

3. Vorrichtung nach Anspruch 1 und 2, dadurch gekennzeichnet, daß der Prothesenbefestigungsüberzieher (17) in seiner Zwischenzone (17c) eine doppelte Dehnbarkeit aufweist, nämlich eine relativ geringe in axialer Richtung (19) und einer stärker dehnbare in radialer Richtung (20) in der Höhe des Gelenkes des gestützten Gliedmaßes.

4. Vorrichtung nach einem der Ansprüche 1 bis 3, dadurch gekennzeichnet, daß der Prothesenbefestigungsüberzieher (17) in seiner Zwischenzone (17c) auf seiner hinteren Mantellinie (21) in der Höhe der Falten für das Gelenk eine das Falten fördernde Vergrößerung des Durchmessers (22) aufweist.

5. Vorrichtung nach einem der Ansprüche 1 bis 4, dadurch gekennzeichnet, daß der Prothesenbefestigungsüberzieher (17) auf der gesamten Länge seiner hinteren Mantellinie (21) ein nahezu nicht ausdehnbares Band (23) aufweist, das die Funktion hat, die Größe einer Streckung in Vorwärtsrichtung bei einer Gehbewegung zu begrenzen.

6. Vorrichtung nach einem der Ansprüche 1 bis 5, dadurch gekennzeichnet, daß der Prothesenbefestigungsüberzieher (17) am Rand seiner oberen Öffnung einen Erkennungswulst aufweist.

**Claims**

1/ A prosthesis fixing device for protecting and fixing an amputated limb (10) in a prosthesis (11) having a removable sleeve (12), the top end of the sleeve (12) which is inserted in the prosthesis and which projects from the top of the prosthesis (11) constituting the rim (11a) of the top limb-receiving opening of the prosthesis (11), said device comprising a first fine lining (13) for coming into contact with the amputated limb (10) and covered with a stump-covering sock (14), and being characterized in that it further comprises a second fine lining (15) covering the sock and having a low coefficient of friction for coming into contact with the sleeve (12), and a third fine lining (16) for covering the sleeve and having a low coefficient of friction for coming into contact with the inside of the amputated limb-receiving cavity of the prosthesis (11), and a prosthesis-fixing tube (17) of elastic cloth and open at both ends surrounding both the prosthesis (11) and the protected limb (10), and having a top elastic fixing zone (17a) and a bottom elastic fixing zone (17b) in the vicinity of its two open ends respectively together with an intermediate zone (17c) of different elastic properties, said prosthesis-fixing tube (17) partially covering the top portion of the limb-contacting first fine lining (13), the sock (14), the sock-covering second fine lining (15) and the sleeve-covering third fine lining (16), said top portions projecting beyond the top zone (17a) of the prosthesis-fixing tube (17) by an amount such that they may be folded down a first time in a single fold over the end of the top zone (17a) of the prosthesis-fixing tube (17) while leaving enough length for forming a second fold by being folded together with said end of said top zone (17a) in order to form a flat elastic fixing wad whose bottom edge (13a) abuts against the rim (11a) of the top limb-receiving opening of the prosthesis (11).

2/ A device acording to claim 1, characterized in that both ends (17a, 17b) of the prosthesis-fixing elastic tube (17) are inextensible axially while being extensible radially.

3/ A device according to claims 1 and 2, characterized in that the intermediate zone (17c) of the prosthesis-fixing elastic tube (17) is extensible in two directions, having relatively low extensibility in the axial direction (19) and relatively high extensibility in the radial direction (20) level with the joint in the protected limb.

4/ A device according to any one of claims 1 to 3, characterized in that the intermediate zone (17c) of the prosthesis-fixing tube (17) is of increased diameter (22) at its rear generator line (21) level with the joint fold, thereby facilitating folding.

5/ A device according to any one of claims 1 to 4, characterized in that the prosthesis-fixing tube (17) has a practically inextensible strip (23) running along the entire length of its rear generator line (21) in order limit the amplitude of extension in the rear-front direction while walking.

6/ A device according to any one of claims 1 to 5, characterized in that the prosthesis-fixing tube (17) has a thickening (24) marking the edge of its top opening.

**FIG : 1**

**FIG:2**

**FIG:3**

**FIG:4**